# FASCICULE DE BREVET EUROPEEN

(11) **EP 2 142 171 B1**
(45) Date de publication et mention de la délivrance du brevet: **07.09.2011**
(21) Numéro de dépôt: 08749588.3
(22) Date de dépôt: 17.04.2008
(51) Int. Cl.: A61K 9/12, A61K 47/10, A61K 8/04, A61P 17/00

(54) **MOUSSE ANTIFONGIQUE A BASE DE CICLOPIROXOLAMINE ET DE PYRITHIONE DE ZINC ET SES APPLICATIONS MEDICALES ET COSMETIQUES**
ANTIMYKOTISCHER SCHAUM MIT CICLOPIROXOLAMIN UND ZINKPYRITHION SOWIE MEDIZINISCHE UND KOSMETISCHE ANWENDUNG DAFÜR
ANTIFUNGAL FOAM CONTAINING CICLOPIROXOLAMINE AND ZINC PYRITHIONE, AND MEDICAL AND COSMETIC APPLICATIONS THEREOF

(30) Priorité: 18.04.2007 FR 0754558
(43) Date de publication de la demande: 13.01.2010
(73) Titulaire: Pierre Fabre Dermo-Cosmétique, 92100 Boulogne-Billancourt (FR)
(72) Inventeur: JEANJEAN, Michel, F-31320 Castanet Tolosan (FR); SENEGAS, Nadine, F-31320 Castanet Tolosan (FR)
(74) Mandataire: Ahner, Francis
(86) Numéro de dépôt international: PCT/EP2008/054657
(87) Numéro de publication internationale: WO 2008/135361

(56) Documents cités:
- EP-A- 0 680 745
- WO-A-94/16710
- WO-A-2005/115336
- WO-A-2008/076416
- US-A- 5 798 093
- US-A1- 2004 241 099
- US-A1- 2005 238 597
- US-A1- 2006 269 485
- US-B1- 6 284 234
- C. ROQUES ET AL.: "In vitro antifungal efficacy of ciclopiroxolamine alone and associated with zinc pyrithione compared to ketoconazole against Malassezia globosa and Malassezia restricta reference strains" MYCOPATHOLOGIA, vol. 162, 2006, pages 395-400, XP019452935

## Description

L'invention concerne un produit se présentant sous forme de mousse destinée au traitement de la dermatite séborrhéique du cuir chevelu et de certaines zones du visage.

La dermatite séborrhéique est une dermatose de l'adulte fréquente mais bénigne, et qui touche principalement le cuir chevelu et le visage. Elle a néanmoins des conséquences importantes sur la qualité de vie des personnes qui en souffrent, induisant ainsi une demande thérapeutique importante.

La dermatite séborrhéique est d'intensité extrêmement variable, si bien que le tableau clinique s'étale de l'état pelliculaire simple jusqu'aux formes les plus sévères où les lésions confluent en plaques pouvant recouvrir la totalité du cuir chevelu.

Des démangeaisons associées à une sensation de brûlure superficielle en constituent les premiers symptômes.

Au stade banal, dit état pelliculaire sec, les squames sont fines, blanchâtres et très peu adhérentes. Elles viennent saupoudrer les vêtements au niveau des épaules.

Dans sa forme typique, dite état pelliculaire gras, les squames deviennent plus épaisses et importantes, parfois sous forme de croûtes. Un érythème diffus apparaît sous les squames, et devient visible sur les bords externes des lésions, comme le front ou les tempes.

Dans les formes les plus sévères, les lésions peuvent recouvrir la totalité du cuir chevelu. A ce stade, la dermatite séborrhéique devient très inflammatoire avec apparition d'un prurit, de squames grasses et épaisses pouvant émettre une odeur désagréable.

La thérapeutique repose principalement sur le traitement symptomatique des zones atteintes.

Dans le cas de la dermatite séborréhique, les démangeaisons associées à l'état pelliculaire du cuir chevelu sont la plupart du temps dues à la prolifération massive de la levure appelée *Malassezia.*

Dans les conditions normales, *Malassezia* se retrouve sur la peau chez 90% des adultes sains et se localise préférentiellement sur le cuir chevelu, les conduits auditifs externes, le visage et les zones médianes du dos et de la poitrine.

Son caractère lipohile explique sa localisation préférentielle sur les zones riches en glandes sébacées, et sa keratinophilie explique l'absence de lésions au niveau des muqueuses.

*Malassezia* peut être responsable d'un certain nombre de pathologies, comme le *Pityriasis versicolor,* les folliculites pityrosporiques, la pustulose néonatale...

Dans le cas de la dermatite séborrhéique, l'action de *Malassezia* s'exerce vraisemblablement par un mécanisme immunologique pro-inflammatoire.

Le traitement, essentiellement local, pourra reposer sur 2 types d'activités agissant complémentairement sur la symptomatologie : une activité anti-inflammatoire d'une part, et une activité antifongique d'autre part.

Dans le cadre de l'invention, trois composants ont été associés :
1. La ciclopiroxolamine, qui a été utilisée pour son action rapide sur les levures de type *Malassezia.*
2. La pyrithione de zinc, qui permet d'inhiber la prolifération des levures et normalise le phénomène de desquamation. Elle contribue également à calmer les démangeaisons.
3. Un agent mouillant, dont l'action mécanique mouillante sur les squames permet leur élimination rapide. Il agit en induisant le gonflement et l'éclatement des substances cémentantes entraînant ainsi la dispersion des squames en fines particules. L'intérêt d'associer la ciclopiroxolamine à la pyrithione de zinc dans le traitement de la dermatite séborrhéique a été démontrée cliniquement et a fait l'objet de publications. Une synergie entre ces deux principes actifs a en effet été constatée, les dilutions actives de l'association étant deux à huit fois supérieures à celles des principes actifs seuls. Par, exemple dans l'article scientifique de Christine Roques et al., Mycopathologia (2006), 162: 395-400, l'activité antifongique de la ciclopiroxolamine seule et associée avec du pyrithione de zinc, a été comparée de manière *in vitro* au ketoconazole contre des souches références de *Malassezia globosa* et *Malassezia restricta.* Les résultats divulgués montrent un effet synergique de la ciclopiroxolamine associée avec du pyrithione de zinc sur le contrôle de la croissance et la réduction des populations de Malassezia.

Les produits actuellement sur le marché sont essentiellement des shampooings, dont le temps de contact trop bref avec le cuir chevelu pénalise fortement l'efficacité. De plus, l'activité détergente propre au shampooing ne permet pas toujours une utilisation fréquente en raison du risque d'emballement du cycle sébacé.

A cela s'ajoute le mal vécu des patients atteints de dermatite séborrhéique, qui ont besoin d'un traitement adapté à la chronicité de la pathologie.
Or, dans l'état de la technique, il est connu qu'il existe des compositions pharmaceutiques topiques contenant des principes actifs qu'ils soient antihistaminiques (US 2005/0238597) ou anti-hyperalgésiques (US 5798093), contenant éventuellement des antifongiques.
Il est également connu d'ajouter un antifongique à un antibactérien dans une composition topique, que ce soit afin de limiter la chute des cheveux (EP 0680745) ou pour le traitement de maladies inflammatoires de la peau (WO 2005/115336).
Il est en outre possible de promouvoir la pénétration transmembranaire d'agents actifs (antipelliculaire, de promotion de croissance des cheveux, etc.) en utilisant certains lipides/surfactants non-ioniques (US 6284234 B1).

L'objet de l'invention est donc de proposer une forme topique permettant un usage très fréquent, voire quotidien de celle-ci.

La forme mousse a été retenue comme étant la plus adaptée pour plusieurs raisons.

Tout d'abord, elle permet l'augmentation du temps de contact des principes actifs avec la peau, donc leur pénétration dans l'espace pilo-sébacé.

Contrairement au shampooing dont l'élimination est quasi immédiate par rinçage, la mousse présente un pouvoir de pénétration beaucoup plus important du fait de sa nature lipophile donc de son affinité pour le cuir chevelu également lipophile. Par ailleurs du fait de l'évaporation de l'alcool du propulseur, le produit se trouve concentré sur le cuir chevelu.

Aussi, en raison de sa forme expansée, la mousse est d'une répartition facile et permet d'éviter toute surcharge locale sur le cheveu liée à un surdosage lors de l'application.

Afin d'éviter tout risque d'alourdissement inesthétique du cheveu, la base de la mousse doit privilégier les composés volatils. Ces derniers assurent d'une part une bonne dissolution des principes actifs, facilitant leur pénétration, et d'autre part ils ne laissent pas de dépôt indésirable sur le cheveu.

La forme aérosol permet de stabiliser la composition en la tenant à l'abri de toute oxydation.
Des compositions pharmaceutiques topiques de type « mousse » sont décrites dans la demande de brevet US 2004/0241099A1 dans le cadre de plusieurs applications thérapeutiques de maladies de peau (eczéma, dermatite atopique, psoriasis, tinea pedis, etc).
En outre, il est divulgué dans WO 94/16710 des compositions topiques contenant un antifongique : le kétokonazole, un glucocorticosteroides d'acétonide et un véhicule acceptable du point de vue dermatologique.

Toutefois, il n'existe à ce jour aucune base d'excipients susceptible de véhiculer de façon stable et acceptable deux principes actifs antifongiques tels que la pyrithione de zinc et la ciclopiroxolamine.

En raison de leurs caractéristiques physico-chimiques éloignées, la pyrithione de zinc et la ciclopiroxolamine présentent en effet des comportements différents, entraînant des contraintes galéniques importantes pour les formuler dans une base commune.

La pyrithione de zinc se présente sous forme de fine suspension particulaire dont la granulométrie est à plus de 95% inférieure à 2 microns. Cette finesse permet de l'utiliser en formule aérosol sans risque de boucher les canaux de distribution de la valve ou du diffuseur de la bombe aérosol. Sa densité très supérieure à l'eau (voisine de 1,7) rend difficile la stabilisation de sa suspension dans le milieu.

La ciclopiroxolamine est un hétérocycle organique appartenant à la famille des N-hydroxypyridones. Lorsqu'elle est concentrée à 1% elle nécessite des teneurs volumiques en alcool comme l'éthanol ou l'isopropanol voisines de 35%, associées à un polyol comme l'hexylène glycol ou l'hexane-1,6-diol, pour être parfaitement solubilisée dans une base émulsionnée.

Or, il est bien connu et techniquement prouvé, qu'une teneur en alcool aussi élevée constitue un élément déstabilisant des systèmes émulsionnés.

Par ailleurs, de telles quantités d'alcool modifient les tensions de surface des tensioactifs, se traduisant par une difficulté à expanser une émulsion sous forme de mousse par le biais d'un aérosol, notamment un aérosol pressurisé à propulseur liquide.

La demanderesse a obtenu de manière surprenante une émulsion stable conditionnée en boîtier aérosol, renfermant une quantité importante d'alcool, et pouvant néanmoins être expansée sous forme de mousse, notamment en présence de gaz liquéfié.

Plus précisément, l'invention concerne une composition sous forme d'émulsion pouvant être expansée sous forme de mousse par un distributeur aérosol, caractérisée en ce qu'elle comprend de 15 à 50% en volume d'alcool, de 1 à 10% en volume d'un polyol en C₃ à C₈, un agent épaississant, au moins un tensioactif non ionique, un agent mouillant et plusieurs principes actifs dissous ou en suspension, selon la présente revendication 1.

On entend par "mousse" une substance formée par l'emprisonnement de bulles de gaz dans un liquide ou un solide.

On entend par "distributeur aérosol" tout flacon pressurisé permettant de dispenser une composition sous forme aérosol ou sous forme de mousse. A titre d'exemple on citera les distributeurs aérosol à gaz liquéfié, ainsi que les distributeurs aérosol à pompe mécanique, appelés couramment "bombe aérosol".

On entend par "alcool" un alkanol comprenant de 2 à 4 atomes de carbone, à chaine linéaire ou ramifiée, comme par exemple l'éthanol ou l'isopropanol.

On entend par "agent épaississant" toute substance viscosante ou texturante, pouvant être de nature polymérique. On citera l'argile à titre d'exemple.

On entend par "agent mouillant" une substance qui permet la dispersion particulaire dans un milieu. L'agent mouillant a ici pour rôle de disperser la substance en suspension, comme la pyrithione de zinc.

On entend par "polyol en C₃ à C₈" un composé à chaîne hydrocarbonée linéaire, ramifiée ou cyclique, comprenant de 3 à 8 atomes de carbone, substituée par au moins deux groupes hydroxyle. On citera à titre d'exemple le propanediol, l'hexylène glycol ou l'hexane-1,6-diol.

De façon avantageuse, la composition comprend de 20 à 40% en volume d'alcool, plus avantageusement 35%.

L'alcool est avantageusement choisi parmi l'éthanol et l'isopropanol.

De façon avantageuse, la composition comprend de 1 à 8% en volume de polyol en C₃ à C₈, plus avantageusement encore de 1 à 5%.

Pour assurer la stabilisation de la suspension au sein de l'émulsion, il est nécessaire de faire appel à des polymères de nature hydrophile capables de former des réseaux en phase aqueuse et bloquer les phénomènes de sédimentation particulaire. En effet, l'usage de ce type de polymère est rendu indispensable en raison de la fluidité de l'émulsion utilisée dans l'invention décrite. Ils jouent donc le rôle d'agent épaississant.

L'agent épaississant peut être avantageusement choisi parmi les polysaccharides d'origine végétale, comme la gomme xanthane et ses dérivés, les copolymères d'alkylacrylates et d'acides acrylique et méthacrylique, notamment obtenus par voie de synthèse, et les silicates, notamment les silicates d'origine minérale plus communément connus sous le nom de Veegum® ou Bentones®.

La proportion en poids d'agent épaississant est avantageusement comprise entre 0,1 et 1% du poids total de la composition, plus avantageusement entre 0,2 et 0,7%.

Le au moins un tensioactif non ionique peut être avantageusement choisi parmi les tensioactifs cireux ou liquides de façon à écarter tout risque d'interaction chimique avec les principes actifs et l'émulsion.

La composition peut notamment comprendre un mélange de tensioactifs non ioniques binaire ou ternaire.

Le au moins un tensioactif non ionique peut être choisi parmi les esters éthoxylés, les mélanges d'alcools gras, les alcools gras éthoxylés en C₁₂ à C₁₈, les triglycérides hydrogénés et éthoxylés, les alkyloxydes d'amines, les diéthanolamides.

La proportion en poids de tensioactif est avantageusement comprise entre 1 et 30% du poids total de la composition, plus avantageusement entre 1 et 15%, plus avantageusement entre 1 et 10%, plus avantageusement comprise entre 2 et 8%, plus avantageusement encore égale à 6%.

Les esters éthoxylés peuvent être choisis parmi le monolaurate de sorbitan éthoxylé (20EO), le monopalmitate de sorbitan éthoxylé (20EO), le monostéarate de sorbitan éthoxylé (20EO), le peroléate de sorbitan éthoxylé (40EO) et le cocoate de glycéryle éthoxylé (7EO). Le chiffre précédant EO désigne le nombre d'unités éthylène oxyde.

Les mélanges d'alcools gras peuvent être choisis parmi le ceteareth-20 (20EO), le ceteareth-33 (33EO) et le ceteareth-50 (50EO).

Les alcools gras éthoxylés en C₁₂ à C₁₈ peuvent être avantageusement choisis parmi le laureth-4 (alcool laurique 4EO) et l'oleth-10 (alcool oléique 10EO).

Le triglycéride hydrogéné et éthoxylé est avantageusement l'huile de ricin hydrogénée éthoxylée 40EO, comme par exemple le produit connu sous la marque Crémophor RH40 de BASF.

L'alkoxyde d'amine est avantageusement la stéaryldiméthylaminoxyde, connue sous la marque Amoxyx SO.

La diéthanolamide peut être choisie parmi l'isostéaramide DEA (isostéaramide diéthanolamine) et la cocamide MEA (cocamide monoéthano lamine).

Avantageusement, les tensioactifs peuvent être choisis parmi les mélanges binaires ou ternaires suivant :
1. Huile de ricin hydrogénée éthoxylée (40EO)
   Monostéarate de sorbitan (20EO)
2. Alcool oléique éthoxylé (10EO)
   Mélange d'alcools cétylique et stéarylique (50EO)
3. Stéaryldiméthylaminoxyde
   Alcool laurique éthoxylé (4EO)
   Peroléate de sorbitan (40EO)
4. Ceteareth (50EO)
   Monolaurate de sorbitan (20EO)
   Peroléate de sorbitan (40EO)
5. Monopalmitate de sorbitan éthoxylé (20EO)
   Alcool oléique éthoxylé (10EO)
   Triglycérides de coco éthoxylés
6. Alcool oléique éthoxylé (10EO)
   Cocoate de glycéryle éthoxylé (7EO)
   Huile de ricin hydrogénée éthoxylée.

Tous ces tensioactifs sont décrits dans l'ouvrage intitulé "International Cosmetic Ingrédient Dictionnary and Handbook", 11ème édition, 2006.

Avantageusement, la composition selon l'invention comprend de l'alcool oléique éthoxylé à une concentration en poids comprise entre 1 et 10% du poids total de la composition, du cocoate de glycéryle éthoxylé à une concentration en poids comprise entre 1 et 10% du poids total de la composition, de l'huile de ricin hydrogénée éthoxylée à une concentration en poids comprise entre 1 et 10% du poids total de la composition.

Avantageusement, la composition selon l'invention comprend de l'alcool oléique éthoxylé à une concentration en poids comprise entre 1 et 5% du poids total de la composition, du cocoate de glycéryle éthoxylé à une concentration en poids comprise entre 1 et 5% du poids total de la composition, de l'huile de ricin hydrogénée éthoxylée à une concentration en poids comprise entre 1 et 5% du poids total de la composition.

L'agent mouillant est de préférence l'acétamide MEA (acétamide monoéthano lamine) ou le lactamide MEA (lactamide monoéthanolamine).

La proportion en poids d'agent mouillant est avantageusement comprise entre 0,1 et 5% du poids total de la composition, plus avantageusement comprise entre 1 et 4%, plus avantageusement encore égale à 2%.

Les principes actifs présents dans les compositions selon l'invention sont la ciclopiroxolamine et la pyrithione de zinc, avantageusement à une proportion en poids comprise entre 0,5 et 2% du poids total de la composition pour chacun des principes actifs, de préférence égale à 1% pour chacun des principes actifs.

La composition selon l'invention peut être conditionnée en distributeur aérosol, notamment en distributeur aérosol à gaz liquéfié.

Les compositions selon l'invention pourront être avantageusement formulées comme suit.

| **Ingrédients** | **Quantité (g)** |
|---|---|
| Ciclopiroxolamine | 0,5-2 |
| Pyrithione de zinc | 0,5-2 |
| Acétamide MEA | 0,1-5 |
| Polymère réticulé | 0,1-1 |
| d'acrylates/d'acrylate alkyle C10-C30 | |
| Triéthanolamine | 0,1-1 |
| Alcool oléique éthoxylé | 1-10 |
| Cocoate de glycéryle éthoxylé | 1-10 |
| Huile de ricin hydrogénée éthoxylée | 1-10 |
| Ethanol | 15-40 |
| Hexylène glycol | 1-10 |
| Eau déminéralisée | q.s.p. 100 |

Les gaz liquéfiés sont ceux classiquement utilisés comme par exemple le diméthyle éther, les hydrofluoroalkanes et les mélanges binaires ou ternaires d'hydrocarbures choisis parmi le butane, le propane ou l'isobutane.

L'invention a également pour objet les compositions telles que décrites précédemment en tant que médicament ou en tant que cosmétique.

L'invention va maintenant être illustrée par l'exemple suivant.

Une composition selon l'invention, contenant de la ciclopiroxolamine et de la pyrithione de zinc, a été formulée comme suit :

| **Ingrédients** | **Quantité (g)** |
|---|---|
| Ciclopiroxolamine | 1 |
| Pyrithione de zinc | 1 |
| Acétamide MEA | 2 |
| Polymère réticulé | 0,6 |
| d'acrylates/d'acrylate alkyle C10-C30 | |
| Triéthano lamine | 0,5 |
| Alcool oléique éthoxylé | 0,5 |
| Cocoate de glycéryle éthoxylé | 3 |
| Huile de ricin hydrogénée éthoxylée | 2,5 |
| Ethanol | 30 |
| Hexylène glycol | 3 |
| Eau déminéralisée | q.s.p. 100 |

Elle a ensuite été conditionnée en aérosol, à raison de 90 g pour 10 g d'un mélange de butane, de propane et d'isobutane.

La composition ci-dessus génère lors de son utilisation une mousse abondante qui s'applique et se répartit aisément sur le cuir chevelu en provoquant un effet apaisant immédiat, puis se rince facilement.

Des études ont montré que la composition ci-dessus permettait une nette diminution des signes cliniques associés à la dermatite séborrhéique (érythème et prurit), dès la première semaine de traitement.

Après 29 jours de traitement, plus de 65% des patients ont été guéris de la dermatite séborrhéique et plus de 34% ont vu leur état nettement s'améliorer.

Après quatre semaines d'utilisation, les effets suivants ont été observés pour la quasi-totalité des patients : un apaisement du cuir chevelu, une diminution des rougeurs, des pellicules, des squames et du regraissage des cheveux.

## Revendications

1. Composition sous forme d'émulsion pouvant être expansée sous forme de mousse par un distributeur aérosol, ladite composition comprenant de 15 à 50% en volume d'un alkanol comprenant de 2 à 4 atomes de carbone, de 1 à 10% en volume d'un polyol en C₃ à C₈, un agent épaississant, au moins un tensioactif non ionique, un agent mouillant et des principes actifs qui sont la pyrithione de zinc et la ciclopiroxolamine dissous ou en suspension, de préférence à une proportion en poids comprise entre 0,5 et 2% du poids total de la composition pour chacun des principes actifs, de préférence égale à 1 % pour chacun des principes actifs.

2. Composition selon la revendication 1, **caractérisée en ce qu'**elle comprend 35% en volume d'alkanol.

3. Composition selon la revendication 1 ou 2, **caractérisée en ce que** l'alkanol est choisi parmi l'éthanol et l'isopropanol.

4. Composition selon l'une quelconque des revendications précédentes, **caractérisée en ce que** l'agent épaississant est choisi parmi les polysaccharides d'origine végétale, de préférence la gomme de xanthane et ses dérivés, les copolymères d'alkylacrylates et d'acides acrylique et méthacrylique et les silicates.

5. Composition selon l'une quelconque des revendications précédentes, **caractérisée en ce que** le au moins un tensioactif non ionique est choisi parmi les tensioactifs cireux ou liquides.

6. Composition selon l'une quelconque des revendications précédentes, **caractérisée en ce que** le au moins un tensioactif non ionique est choisi parmi les esters éthoxylés, les mélanges d'alcools gras, les alcools gras éthoxylés en C₁₂ à C₁₈, les triglycérides hydrogénés et éthoxylés, les alkyloxydes d'amines, les diéthanolamides.

7. Composition selon la revendication précédente, **caractérisée en ce que** les esters éthoxylés sont choisis parmi le monolaurate de sorbitan éthoxylé, le monopalmitate de sorbitan éthoxylé, le monostéarate de sorbitan éthoxylé, le peroléate de sorbitan éthoxylé et le cocoate de glycéryle éthoxylé.

8. Composition selon la revendication 6, **caractérisée en ce que** les mélanges d'alcools gras sont choisis parmi le ceteareth-20, le ceteareth-33 et le ceteareth-50.

9. Composition selon la revendication 6, **caractérisée en ce que** les alcools gras éthoxylés en C₁₂ à C₁₈ sont choisis parmi le laureth-4 et l'oleth-10.

10. Composition selon la revendication 6, **caractérisée en ce que** le triglycéride hydrogéné et éthoxylé est l'huile de ricin hydrogénée éthoxylée.

11. Composition selon la revendication 6, **caractérisée en ce que** l'alkoxyde d'amine est la stéaryldiméthylaminoxyde.

12. Composition selon la revendication 6, **caractérisée en ce que** la diéthanolamide est choisie parmi l'isostéaramide DEA et la cocamide MEA.

13. Composition selon l'une quelconque des revendications précédentes, **caractérisée en ce qu'**elle comprend de l'alcool oléique éthoxylé à une concentration en poids comprise entre 1 et 10% du poids total, du cocoate de glycéryle éthoxylé à une concentration en poids comprise entre 1 et 10% du poids total, de l'huile de ricin hydrogénée éthoxylée à une concentration en poids comprise entre 1 et 10% du poids total.

14. Composition selon l'une quelconque des revendications précédentes, **caractérisée en ce que** l'agent mouillant est l'acétamide MEA ou le lactamide MEA.

15. Composition selon l'une quelconque des revendications précédentes, **caractérisée en ce qu'**elle présente la composition centésimale suivante :
| | |
|---|---|
| Ciclopiroxolamine | 0,5-2 |
| Pyrithione de zinc | 0,5-2 |
| Acétamide MEA | 0,1-5 |
| Polymère réticulé d'acrylates/d'acrylate alkyle C10-C30 | 0,1-1 |
| Triéthanolamine | 0,1-1 |
| Alcool oléique éthoxylé | 1-10 |
| Cocoate de glycéryle éthoxylé | 1-10 |
| Huile de ricin hydrogénée éthoxylée | 1-10 |
| Ethanol | 15-40 |
| Hexylène glycol | 1-10 |
| Eau déminéralisée | q.s.p. 100 |

16. Composition selon l'une quelconque des revendications précédentes, **caractérisée en ce qu'**elle est conditionnée en distributeur aérosol.

17. Composition selon l'une quelconque des revendications précédente, pour son utilisation pour le traitement de la dermite sébohrréique du cuir chevelu et de certaines zones du visage.

## Claims

1. Composition in the form of an emulsion that can be expanded using an aerosol dispenser to generate a foam, said composition containing 15 - 50% by volume of an alkanol comprising 2 to 4 carbon atoms, 1 to 10% by volume of a C₃ - C₈ polyol, a thickening agent, at least one non-ionic surfactant, a wetting agent, and active substances, namely zinc pyrithione and ciclopiroxolamine dissolved or in suspension, preferably comprising each active substance at a weight proportion of between 0.5% and 2% of the total weight of the composition, and more preferably still equal to 1% for both active substances.

2. Composition according to Claim 1, **characterised in that** it contains 35% alcohol by volume.

3. Composition according to Claim 1 or 2, **characterised in that** the alkanol is chosen from ethanol or isopropanol.

4. Composition according to any of the previous Claims, **characterised in that** the thickening agent is selected from plant-derived polysaccharides, preferably xanthane gum and its derivatives, copolymers of alkylacrylates and of acrylic and methacrylic acids, and silicates.

5. Composition according to any of the previous Claims, **characterised in that** said at least one non-ionic surfactant is selected from the group of waxy or liquid surface active.

6. Composition according to any of the previous Claims, **characterised in that** said at least one non-ionic surfactant is selected from ethoxylated esters, fatty alcohol mixtures, ethoxylated C₁₂ - C₁₈ fatty alcohols, hydrogenated and ethoxylated triglycerides, amine alkoxides, or diethanolamides.

7. Composition according to the previous Claim, **characterised in that** the ethoxylated esters are selected from the group of ethoxylated sorbitan monolaurate, ethoxylated sorbitan monopalmitate, ethoxylated sorbitan monostearate, ethoxylated sorbitan peroleate and ethoxylated glyceryl cocoate.

8. Composition according to Claim 6, **characterised in that** the mixtures of fatty alcohols are selected from the group of ceteareth-20, ceteareth-33 and ceteareth-50.

9. Composition according to Claim 6, **characterised in that** the ethoxylated C₁₂ - C₁₈ fatty alcohols are selected from laureth-4 and oleth-10.

10. Composition according to Claim 6, **characterised in that** the hydrogenated and ethoxylated triglyceride is hydrogenated and ethoxylated castor oil.

11. Composition according to Claim 6, **characterised in that** the amine alkoxide is stearyl dimethylaminoxide.

12. Composition according to Claim 6, **characterised in that** the diethanolamide is either isostearamide DEA or cocamide MEA.

13. Composition according to any of the previous Claims, **characterised in that** it contains ethoxylated oleic alcohol at a weight proportion comprised between 1% and 10% of the total weight, ethoxylated glyceryl cocoate at a weight proportion comprised between 1% and 10% of the total weight, hydrogenated and ethoxylated castor oil at a weight proportion comprised between 1% and 10% of the total weight.

14. Composition according to any of the previous Claims, **characterised in that** the wetting agent is either acetamide MEA or lactamide MEA.

15. Composition according to any one of the previous Claims, **characterised in that** it has the following percentage composition:
| | |
|---|---|
| Ciclopiroxolamine | 0.5 - 2 |
| Zinc pyrithione | 0.5 - 2 |
| Acetamide MEA | 0.1 - 5 |
| Cross-linked polymer of acrylate and C10-C30 alkyl acrylate | 0.1 - 1 |
| Triethanolamine | 0.1 - 1 |
| Ethoxylated oleic alcohol | 1 - 10 |
| Ethoxylated glyceryl cocoate | 1 - 10 |
| Ethoxylated and hydrogenated castor oil | 1 - 10 |
| Ethanol | 15 - 40 |
| Hexylene glycol | 1 - 10 |
| Demineralized water | q.s.100 |

16. Composition according to any of the previous Claims, **characterised in that** it is supplied in an aerosol dispenser.

17. Use of the composition according to any of the previous Claims, to treat seborrhoeic dermatitis of the scalp or certain areas of the face.

## Patentansprüche

1. Zusammensetzung in Form einer Emulsion, welche durch einen Aerosol-Spender in Form eines Schaums aufgeschäumt werden kann, wobei die Zusammensetzung 15 bis 50 Vol.-% eines Alkanols, welches 2 bis 4 Kohlenstoffatome umfasst, 1 bis 10 Vol.-% eines C₃- bis C₈-Polyols, ein Verdickungsmittel, mindestens ein nichtionisches grenzflächenaktives Mittel, ein Benetzungsmittel und Wirkstoffe, die Zinkpyrithion und Cyclopiroxolamin, gelöst oder in Suspension, sind, vorzugsweise in einem Gewichtsanteil zwischen 0,5 und 2% des Gesamtgewichts der Zusammensetzung eingeschlossen für jeden der Wirkstoffe, vorzugsweise von 1 % für jeden der Wirkstoffe umfasst.

2. Zusammensetzung nach Anspruch 1, **dadurch gekennzeichnet, dass** sie 35 Vol.-% Alkanol umfasst.

3. Zusammensetzung nach Anspruch 1 oder 2, **dadurch gekennzeichnet, dass** das Alkanol unter Ethanol und Isopropanol ausgewählt ist.

4. Zusammensetzung nach einem der vorangegangenen Ansprüche, **dadurch gekennzeichnet, dass** das Verdickungsmittel unter den Polysacchariden pflanzlicher Herkunft, vorzugsweise Xanthangummi und dessen Derivate, den Copolymeren von Alkylacrylaten und Acryl- und Methacrylsäuren und den Silicaten ausgewählt ist.

5. Zusammensetzung nach einem der vorangegangenen Ansprüche, **dadurch gekennzeichnet, dass** das mindestens eine nicht-ionische grenzflächenaktive Mittel unter den wachsartigen oder flüssigen grenzflächenaktiven Mitteln ausgewählt ist.

6. Zusammensetzung nach einem der vorangegangenen Ansprüche, **dadurch gekennzeichnet, dass** das mindestens eine nicht-ionische grenzflächenaktive Mittel unter den ethoxylierten Estern, den Mischungen von Fettalkoholen, den ethoxylierten C₁₂- bis C₁₈-Fettalkoholen, den hydrierten und ethoxylierten Triglyceriden, den Aminalkyloxiden, den Diethanolamiden ausgewählt ist.

7. Zusammensetzung nach dem vorangegangenen Anspruch, **dadurch gekennzeichnet, dass** die ethoxylierten Ester unter ethoxyliertem Sorbitanmonolaurat, ethoxyliertem Sorbitanmonopalmitat, ethoxyliertem Sorbitanmonostearat, ethoxyliertem Sorbitanperoleat und ethoxyliertem Glycerylcocoat ausgewählt sind.

8. Zusammensetzung nach Anspruch 6, **dadurch gekennzeichnet, dass** die Mischungen von Fettalkoholen aus Ceteareth-20, Ceteareth-33 und Ceteareth-50 ausgewählt sind.

9. Zusammensetzung nach Anspruch 6, **dadurch gekennzeichnet, dass** die ethoxylierten C₁₂- bis C₁₈-Fettalkohole unter Laureth-4 und Oleth-10 ausgewählt sind.

10. Zusammensetzung nach Anspruch 6, **dadurch gekennzeichnet, dass** das hydrierte und ethoxylierte Triglycerid hydriertes, ethoxyliertes Ricinusöl ist.

11. Zusammensetzung nach Anspruch 6, **dadurch gekennzeichnet, dass** das Aminalkoxid Stearyldimethylaminoxid ist.

12. Zusammensetzung nach Anspruch 6, **dadurch gekennzeichnet, dass** das Diethanolamid unter Isostearamide DEA und Cocamide MEA ausgewählt ist.

13. Zusammensetzung nach einem der vorangegangenen Ansprüche, **dadurch gekennzeichnet, dass** sie ethoxylierten Oleinalkohol in einer auf das Gewicht bezogenen Konzentration zwischen 1 und 10% des Gesamtgewichts eingeschlossen, ethoxyliertes Glycerylcocoat in einer auf das Gewicht bezogenen Konzentration zwischen 1 und 10% des Gesamtgewichts eingeschlossen, hydriertes, ethoxyliertes Ricinusöl in einer auf das Gewicht bezogenen Konzentration zwischen 1 und 10% des Gesamtgewichts eingeschlossen umfasst.

14. Zusammensetzung nach einem der vorangegangenen Ansprüche, **dadurch gekennzeichnet, dass** das Benetzungsmittel Acetamide MEA oder Lactamide MEA ist.

15. Zusammensetzung nach einem der vorangegangenen Ansprüche, **dadurch gekennzeichnet, dass** sie die folgende zentesimale Zusammensetzung aufweist:
| | |
|---|---|
| Cyclopiroxolamin | 0,5-2 |
| Zinkpyrithion | 0,5-2 |
| Acetamide MEA | 0,1-5 |
| Vernetztes Polymer von C₁₀-C₃₀-Acrylaten/-Alkylacrylaten | 0,1-1 |
| Triethanolamin | 0,1-1 |
| Ethoxylierter Oleinalkohol | 1-10 |
| Ethoxyliertes Glycerylcocoat | 1-10 |
| Hydriertes, ethoxyliertes Ricinusöl | 1-10 |
| Ethanol | 15-40 |
| Hexylenglycol | 1-10 |
| Demineralisiertes Wasser | auf 100. |

16. Zusammensetzung nach einem der vorangegangenen Ansprüche, **dadurch gekennzeichnet, dass** sie in einem Aerosol-Spender verpackt ist.

17. Zusammensetzung nach einem der vorangegangenen Ansprüche für ihre Verwendung für die Behandlung der seborrhoischen Dermatitis der behaarten Kopfhaut und von bestimmten Bereichen des Gesichts.
